# EUROPEAN PATENT APPLICATION

(11) **EP 1 693 466 A1**
(43) Date of publication of application: **23.08.2006**
(21) Application number: 05447029.9
(22) Date of filing: 16.02.2005
(51) Int. Cl.: C12Q 1/68, B01L 3/00, G01N 33/574

(54) **Method and kit to profile tumors by biomarker analyses including transcriptional factor assays**

(71) Applicant: Eppendorf Array Technologies SA, B-5000 Namur (BE)
(72) Inventor: Mainfroid, Véronique, 4300 Waremme (BE); Remacle, José, 5020 Malonne (BE)
(74) Representative: Van Malderen, Joëlle

(57) **Abstract**

The present invention is related to a method and kit or device for providing a tumor profile of a biological sample obtained from a tumoral tissue, tumor cells or fluid of a subject, preferably a human subject, by a detection and/or quantification of biomarkers possibly present in the sample.

## Description

### Field of the invention

The present invention is related to a method and kit (or device) for the detection and/or the quantification of biomarkers related to tumorigenesis.

Said method is advantageously used to propose or adapt an anti-tumoral therapeutic protocol to be administered to a subject.

Furthermore, said method and kit (or device) are a technical platform for the identification of new compounds, which are preferably used at (a) specific step(s) of an anti-tumoral therapeutic protocol.

This patent application is a continuation in part of the US patent applications serial numbers 09/816,763 and 10/339,161 from which the content of the description is incorporated herein by reference

### Background of the invention

Despite very sustained and combined efforts by the scientific, medical and pharmaceutical communities to fight cancer, a large proportion of the population within Western countries will develop and succumb to the disease.

Once a tumor has been detected, the standard first-line treatment usually relies on a combination of chemotherapy and radiotherapy. Unfortunately, the success of this therapeutic protocol is mainly limited by the onset of resistance, which is almost inevitable for subjects with advanced disease.

A large body of efforts has been carried out over the past decade to understand the molecular rationale behind this nearly universal problem. They have led to the identification of molecular targets (tumor biomarkers) that may play a major contribution to the adverse effect observed. Associating the presence of these key biomarkers in some tumors and the tumors responsiveness to a particular treatment now offers great hope that tailored treatments to each tumor, for each subject, can soon be proposed.

These prognostic and predictive biomarkers mainly include oncogenes, tumor suppressor genes, angiogenic and anti-angiogenic factors, and proteins involved in proliferation cascades and apoptotic pathways, and many concern transcriptional factors. The identification of such molecular biomarkers has led to the active development by important pharmaceutical companies of many novel active agents, among which several have entered clinical trials. These therapeutic and preventive agents, among which small molecular-weight inhibitors, monoclonal antibodies, antisense oligonucleotides, and gene therapy tools, are being evaluated alone and in combination with cytotoxic chemotherapy. Understanding to which extent a tumor response to a treatment depends on the presence of a biomarker will help predicting the effect of treatment and selecting the most appropriate one for each individual subject.

However, it appears that each cancer type, and even each tumor, has its own biological characteristics. Therefore, a biomarker shown to have a prognostic value for a particular tumor may completely fail to predict the clinical outcome of another tumor. A treatment targeting a biological target will therefore prove efficient only in those tumors whose survival depends on the target.

This is well illustrated with the pro-apoptotic p53, a transcriptional factor known as a tumor suppressor protein, whose activity is deficient in many tumors. The p53 activation status in a tumor has a direct therapeutic consequence, as most first-line chemotherapies rely on cytotoxic molecules, whose efficiency is tributary of functional apoptotic pathways. An increasing number of new therapeutic molecules targeting p53 have therefore emerged as alternatives to the classical treatments. A p53 deficiency can however originate from very different aspects of its molecular biology, and hence, each therapeutic molecule only targets a specific defect. Accordingly, an absence of p53 synthesis may tentatively be compensated by the adenoviral delivery of the corresponding gene; the production by certain tumors of a mutated p53 harboring a modified DNA-binding site may be circumvented by the delivery of small molecular compounds designed to change the structure of the transcriptional factor and restore its DNA-binding capacity; a lack of p53 activity due to the sequestration of the factor, i.e. by the Mdm2 protein, may be opposed through the use of small antagonists interfering with this protein-protein interaction, thereby releasing the factor and restoring its activity.

Making available several pieces of information about the status of hormone-dependent tumors before starting any treatment may also have a high incidence on the success or failure of this treatment.

This is well illustrated with breast cancers, for which the most effective systematic treatment relies on endocrine therapy such as tamoxifen (Novaldex® ) administration. Efficacy, however, relies on the presence of a functional estrogen receptor alpha (ERα), which is almost universally absent in subjects with advanced disease. Moreover, endocrine resistance may also appear in tumors exhibiting a positive ERα expression. The overexpression of ErbB2, a member of a family of four plasma membrane tyrosine kinase receptors, is now recognized as a general player mediating this endocrine resistance. However, at the present time, a simultaneous assessment of both ERα and ErbB2 status in a single assay has not been proposed to predict the potential success of an endocrine therapy. More importantly, subjects harboring ErbB2 positive tumors to whom tamoxifen was administrated were even shown to do worse than those receiving placebo (Hu, J.C.C. & Mokbel, K., Eur. J. Surg. Oncol. 2001, 27: 335-337). The use of ERα or ErbB2 targeting agents should therefore not be prescribed only on the sole expression of the corresponding protein. The situation may even be complicated by the intervention of other players: as an example, endocrine resistance may occur in the absence of any evidence for an ErbB member overexpression. The overexpression -or more precisely overactivation- of a downstream target of the ErbB signaling cascade, which may intersect with the ER pathway, has been suggested. The mammalian target of rapamycin (mTOR), a key intermediate in multiple mitogenic signaling pathways, has been proposed as being such a target. However, the concerted analysis of several biomarkers, such as ERα, ErbB2 and mTOR, in tumors has not been described for prognostic and predictive value.

Ultimately, the success of chemotherapy therefore relies not just on the availability of new therapeutic solutions, but on the ability to identify subjects with biomarkers that may predict their response to these agents. These biomarkers comprise proteins, including DNA binding proteins such as transcriptional factors, or any modification thereof, which may be of predictive value in therapeutic oncology.

Transcriptional factors play a key role as biomarkers, as many evidences now point to one or several transcriptional factors as being central players in the tumorigenic process of many cancers, as already illustrated above with p53 and ERα. However, several aspects of the transcriptional factor biology can take part to the process, pointing to the necessity of simultaneously assessing all of them. Moreover, it appears that the analysis of transcriptional factors, although necessary to understand the tumor development, is not sufficient, as it must be complemented by analyses of one or several (non-transcriptional factor) biomarkers. It is only the overall integration of these analyses that can lead to the successful assessment of a tumor status, and the orientation towards a favorable treatment.

The development of an advanced assay, enabling to simultaneously detect and/or quantify several prognostic biomarkers including transcriptional factors in clinical samples has not been previously described, but would be highly valuable.

### State of the art

The simultaneous detection of several markers associated with a subject's tumor is described in the patent application WO2004/062608. This application describes an assay kit for characterizing a cancer tumor for medical diagnosis and treatment. The assay provides a cancer protein pattern based on detected levels of various biomolecular markers (BMMs) associated with a subject's tumor. The assay is adapted to a multiwell format, where each individual well of the multiwell plate contains one capture protein specific for one of the markers to be detected. Detection is performed by using a detection protein, preferably an antibody.

The patent application US2002/0095073 describes the design and use of diagnostic devices to determine the cause of one or more medical symptoms exhibited by a subject. An array of different probes, each interacting with a target associated with a different known cause of the symptoms, is put in contact with a biological sample, and possible interactions between probes and target molecules present in the biological sample are simultaneously detected and analyzed.

The patent application WO2002/40716 describes a method of diagnosing neoplastic diseases. The presence of neoplastic molecular markers is detected using an array of specific reagents. Based upon the neoplastic molecular marker profile of the subject, the tumor sub-profile is ascertained and an appropriate treatment protocol is initiated.

The patent application WO01/73115 describes a screening, detection and/or quantification method of one or more transcriptional factors possibly present in a biological sample, through their binding to double-stranded DNA sequences bound to an insoluble solid support.

However, none of the methods described above does provide sufficient information to establish a tumor profile of a biological sample obtained from a subject.

In particular, these documents do not provide combined information about the presence and possible modifications of a same protein, and more particularly a transcriptional factor, enabling to select or reject available anti-tumoral therapies.

These methods do not describe nor suggest the combined detection of transcriptional factors, other regulatory proteins and some of their characteristics in a same assay to establish a tumor profile.

### Aims of the invention

The aim of the present invention is to provide a method and kit (or device) for obtaining information that allow a clinician to determine a specific tumor profile of a biological sample obtained from a subject, preferably a human subject by the detection and/or the quantification of biomarkers associated with a type of tumor present in the sample.

A further aim of the invention is to provide such method and kit (or device) for selecting from this tumor profile, the compounds most adapted to treat (or prevent the development of) a tumor affecting this subject, preferably a human subject, to reject an unfavorable treatment, to establish diagnostic and prognostic analyses, to develop and/or test novel compounds with anti-tumoral potential and to follow the efficacy of an anti-tumoral therapy on the subject.

### Summary of the invention

The invention describes a method for providing a tumor profile of a biological sample obtained from a tumoral tissue, tumor cells or a fluid (possibly comprising biomarkers obtained from these tumor cells) of a subject, preferably a human subject, by a detection and/or quantification of tumor biomarkers possibly present in the sample, said method comprising at least the steps of:
a. contacting said biological sample with capture probes,
b. detecting and/or quantifying a first (tumor) biomarker being a transcriptional factor in the biological sample after binding to a first capture probe,
c. detecting and/or quantifying the DNA-binding capacity of the said transcriptional factor in the biological sample after binding to a second capture probe, which possibly differs from the first capture probe and contains a double-stranded DNA sequence specifically binding said transcriptional factor, and
d. detecting and/or quantifying a second (tumor) biomarker different from a transcriptional factor in the biological sample after binding to a third capture probe,
wherein the capture probes are bound directly or indirectly to a solid support, and wherein only the overall integration (or combination) of the results obtained at each step following step (a) provides information on the tumor profile (tumor profile characteristics).

The invention is also related to a diagnostic, quantification and/or screening kit or device comprising means and media for performing the method, which comprises capture probes bound directly or indirectly to one or more solid supports and interacting specifically with biomarkers specific of a tumor profile.

### Detailed description of the invention

The method of the present invention, for providing a tumor profile of a biological sample by a detection and/or quantification of (tumor) biomarkers, is an analytical method particularly suitable for obtaining information of the tumor profile, which means information related to the possible responsiveness of a subject from whom said sample has been obtained to anti-tumoral therapy, especially for selecting one or more adequate anti-tumoral compound(s) to be administered to the subject, preferably a human subject, from whom the biological sample has been obtained.

According to the invention, the biological sample is preferably a protein extract obtained from a tumor sample (which means a biopsy or a biological fluid, such as serum or plasma and therefore possibly comprising these tumor biomarkers. The biological sample used in the present invention can also refer to non-tumoral biological sample that can be taken as a negative control.

The biological sample is also composed of protein extracts from in vitro cultured cells, more particularly protein extracts derived form transformed or immortal cell lines. Transformed or immortal cell lines exhibit some characteristics of tumoral cells and can be part of this invention.

Biological sample also encompasses proteins isolated from any of the biological samples described above following i.e. purification procedures.

The (tumor) biomarkers according to the invention preferably comprise transcriptional factors belonging to the nuclear receptor family, such as the estrogen receptor (ERα), the progesterone receptor (PR) and the androgen receptor (AR), or involved in proliferation and apoptotic cascades, such as the products from oncogenes and tumor suppressor genes, exemplified by cMYC and p53.

The present invention also preferably detects and/or quantifies other biomarkers which are not transcriptional factors. They are preferably involved in intracellular signaling cascades, such as proliferation and apoptosis. The detection of kinases and phosphatases is also a preferred embodiment of the invention. In another preferred embodiment of the invention, the biomarkers are extracellular proteins, such as angiogenic and anti-angiogenic molecules, i.e. VEGF, PlGF, PDGF, bFGF, EGF, HGF, TGFβ, IL-6, IL-8, IL-12, angiopoietin-1, angiopoietin-2, angiogenin, angiostatin, endostatin, thrombospondin-1, matrix metalloproteinases, or mixtures thereof, or cytokines and growth factors.

The present method comprises at least the following steps of:
a. contacting the above described biological sample with capture probes,
b. detecting and/or quantifying a first (tumor) biomarker being a transcriptional factor in the biological sample after binding to a first capture probe,
c. detecting and/or quantifying the DNA-binding capacity of said transcriptional factor in the biological sample after binding to a second capture probe, which possibly differs from the first capture probe and contains a double-stranded DNA sequence specifically binding said transcriptional factor, and
d. detecting and/or quantifying a second (tumor) biomarker different from a transcriptional factor in the biological sample after binding to a third capture probe.

The invention also preferably includes one or both following steps:
e. detecting and/or quantifying a modification of the said transcriptional factor affecting its activity,
f. detecting and/or quantifying the binding capacity of the said transcriptional factor with interacting partner(s), and
g. possibly detecting these interacting partners in the biological sample.

The inventors have found that only the overall integration of the results obtained at each of the successive steps (b) to (d), preferably together with the results of steps (e) (f) and/or (g), provides the necessary and sufficient information regarding the tumor profile and is of high predictive value for subject treatment and for a clinician selecting one or more adequate anti-tumoral compounds and/or adapting a therapeutic or prophylactic anti-tumoral protocol.

Performing only some of the steps (b) to (g) can only generate partial information that is helpful to reject unfavorable therapeutic solutions, but is insufficient to reach the most adequate solution. This is well illustrated in the accompanying examples 1 and 2, where the authors show that if only some of the signals are analyzed, they may take a clinician away from an unfavorable treatment or orient his choice towards one type of therapy, but will not help selecting which molecule of this therapeutic category should be used. Accordingly, the analysis of signals 1 and 2 from example 1 will predict whether a hormonal therapy is adequate or not, but will not inform about an alternative treatment if a hormonal therapy is counter indicated, nor will it help selecting the best compound to be administered if a hormonal therapy is indicated. Hence, the choice between an ERα inhibitor (such as tamoxifen) and an estrogen deprivation therapy (i.e. by administering letrozole) will further depend on the presence or not of a functional ErbB signaling cascade, an information which is only accessible through the analysis of additional signals.

In the present invention, steps (a), (b), (c) and (d) are preferably successive steps. If one transcriptional factor is suspected of being a (tumor) biomarker in a particular sample, the detection and/or quantification of its presence in said sample is a prerequisite to its further analysis. Transcriptional factors exert their biological effect through their binding, in an activated form, to target DNA sequences in the promoter of the genes they regulate. Therefore, the presence of a transcriptional factor in the test sample, assessed at step (b) of the present invention, claims for the further analysis of its DNA-binding capacity through step (c). Step (d), which informs about the presence of a second - non transcriptional factor - tumor) biomarker in the biological sample, is then performed to complete the analysis. In another preferred embodiment of the invention, the successive steps are (a), (d), (b) and (c).

In one particular embodiment of the invention, steps (a), (b), (c) and (d) are complemented with step (e) of detecting and/or quantifying a modification of the transcriptional factor affecting its activity. This is of importance especially for those transcriptional factors which have the particularity of being able to bind DNA in an inactive form. Step (b), which monitors the DNA-binding capacity of the transcriptional factor, is therefore insufficient to attest for the activity of said transcriptional factor, and must be complemented with step (e). This is well illustrated with CREB molecule, a transcriptional factor involved in many physiological functions such as pituitary function, glucose homeostasis, growth factor dependent cell survival, learning and memory. CREB molecule is constitutively bound to DNA, and its activation requires a phosphorylation on its Ser133 residue by specific kinases, such as mitogen activated protein kinases, protein kinase A and Ca2+/calmodulin-dependent protein kinases. In a preferred embodiment of the invention, the modification to be detected at step (e) is a phosphorylation.

In another preferred embodiment of the invention, the modification is an acetylation, as accumulating evidence indicates that acetylation by proteins such as CREB-binding protein (CBP), p300 and P/CAF is a common denominator to many activated transcriptional factors. In another preferred embodiment, the modification is a mutation or a deletion.

In another preferred embodiment, the modification is a conformational change. This is of particular importance for nuclear receptors, as some of them, including ERα, are constitutively bound to DNA and require a change in their tri-dimensional structure to become functionally active. In a preferred embodiment of this invention, the modification is the association of the transcriptional factor with a cofactor, such as a coactivator or a co-repressor. Examples of co-activators and co-repressors are found in Robyr D. et al. (Mol. Endocrinol. 2000, 14(3): 329-347).

In another particular embodiment of the invention, steps (a), (b), (c) and (d), possibly with step (e), are combined with step (f) and possibly (g) of detecting and/or quantifying the binding capacity of a transcriptional factor with interacting partner(s), and possibly detecting these interacting partners in the biological sample. Indeed, if one or several of the transcriptional factors analyzed is/are present in the biological sample but in a complex with interacting partner(s) preventing them from being active, this will highly impact the choice of possible therapeutic compounds. Examples of interactions between transcriptional factors and interacting partner(s) are ERα with AIB1, p53 with Mdm2, p53 with Mdmx, p53 with COP1, HIF1α with p53.

Performing steps (e) and/or (f) and possibly (g) in complement to steps (a), (b), (c) and (d) may highly impact the choice of the treatment. Indeed, some therapeutic compounds or protocols (or association of different therapeutic compounds) have efficient or higher anti-tumoral therapeutic or prophylactic effects only upon an active transcriptional factor. Furthermore, if a transcriptional factor comprises a modification of its activity (for example resulting from one or more mutation(s) or deletions of its amino acids), the activity profile and binding capacity of said (tumor) biomarker to anti-tumoral compounds may be modified accordingly. Similarly, some therapeutic compounds (or association of different therapeutic compounds) targeting said transcriptional factor(s) may have no therapeutic effect if their action is based upon the association with said transcriptional factor at the same binding site already occupied by an interacting partner. Therefore, integrating the information from steps (e) and/or (f) and possibly (g) to those already obtained from steps (a), (b), (c) and (d) can help the clinician to select only some anti-tumoral compounds, and therefore modify the therapeutic or prophylactic protocol applied to the subject.

Therefore, the simultaneous assessment of the presence of a transcription factor (through step b) and its DNA-binding capacity (through step c), possibly in combination with the assessment of modifications affecting its activity (through step e) and its binding capacity to interacting partner(s) (through step f) and possibly step (g) may provide unique information that would not be available through the results obtained from these steps in independent experiments.

The processes of collecting results through steps (a) to (d), possibly with steps (e) and/or (f) and possibly (g) and interpreting results are two parts of a same procedure from this invention. Both parts can be performed by different experimentators, i.e. a lab technician and a clinician, possibly assistance with a programmed computer and the adopted software.

The nucleic acids (DNA) capture probes of the present invention refer to capture probes containing totally or in part double-stranded DNA sequences. They preferably contain a specific sequence of double-stranded DNA linked to a spacer of at least about 6.8 nm, preferably a (DNA) spacer of at least about 20 base pairs, more preferably of at least about 50 base pairs (but lower than about 300 base pairs, preferably lower than about 200 base pairs). Therefore, upon binding the nucleic acids (DNA) capture probes to a solid support, the specific sequence of double-stranded nucleic acids (DNA), which allows binding with corresponding transcriptional factor, is at a certain distance from the surface of the solid support (at least about 6.8 nm) in order to improve the specific detection of said transcriptional factor and its discrimination from other DNA binding proteins.

Nucleic acids (DNA) capture probes are preferentially immobilized through covalent binding of an amino-terminal group onto an activated surface of the support, preferentially bearing (reactive) aldehyde, epoxy or acrylate functions. In another preferred embodiment, nucleic acids (DNA) capture probes are biotinylated at one extremity and are immobilized on an avidin or streptavidin support.

Protein capture probes of the present invention are composed of or contain a protein or peptide moiety. They are preferably (monoclonal) antibodies (or a hypervariable portion thereof) or cofactors such as coactivators, which can be used for a direct and specific binding of the biomarker.

In the method according to the invention, the steps (b), (d) and possibly (f) and/or (g) of detecting the presence of a transcriptional factor, a second (tumor) biomarker and (a) transcriptional factor interacting partner(s) in the biological sample, are preferably obtained by the specific binding of the transcriptional factor, the second (tumor) biomarker or the interacting partner(s) to protein capture probes. In a more preferred embodiment, the protein capture probes are (monoclonal) antibodies or hypervariable portions thereof or receptors.

In the method according to the invention, the step (f) and/or (g) of detecting and/or quantifying the binding capacity of the transcriptional factor with interacting partners is also preferably performed by using DNA capture probes. Contacting the biological sample with the DNA capture probes will result in the binding of the transcriptional factor as a complex with its interacting partner(s) to the DNA capture probes.

In the method according to the invention, the step (c) of detecting and/or quantifying the DNA binding capacity of a transcriptional factor is preferably performed according to the method described in the patent application WO01/73115 incorporated herein by reference. In this preferred embodiment, the DNA capture probe contains a specific sequence, preferably comprised between 15 and 50 base pairs, preferably comprised between 20 and 40 base pairs) of double-stranded DNA bound upon the solid support surface through a spacer having a length of at least 6.8 nm. In a more preferred embodiment, the spacer is composed of or contains a double-stranded DNA sequence (as above described).

In the method according to the invention, the steps (e) and possibly (g) of detecting a modification of the transcriptional factor possibly affecting its activity or detecting the interacting partner(s) are preferably performed by the use of capture probes specifically binding the modified transcriptional factor or the interacting partner(s).

In a more preferred embodiment, the capture probe is an (monoclonal) antibody or a hypervariable portion thereof. In a particular embodiment, the antibodies or hypervariable portions thereof specifically recognize the modified transcriptional factor. In another preferred embodiment, the capture probe is a DNA capture probe if the transcriptional factor modification does not affect its DNA binding site.

Preferably, the methods according to the invention are performed in order to obtain a detection of a specific signal, preferably using non-radioactive detection means. More preferably, each binding between a capture probe and a (tumor) biomarker results in a detectable signal. In a preferred embodiment, said detectable signal is obtained by using specific detection molecules that recognize the (tumor) biomarkers bound to capture probes, more preferably the complexes formed between DNA capture probes and transcriptional factors or between protein capture probes, preferably first (monoclonal) antibodies or hypervariable portions thereof, and these (tumor) biomarkers, including transcriptional factors. Said detection molecules are preferably second (monoclonal) antibodies or hypervariable portions thereof being directly or indirectly labeled. Said label present on the detection molecule results in a detectable signal, being preferably obtained from a chemoluminescent, bioluminescent, fluorescent, colorimetric, radioactive, electronic or magnetic label. Colorimetric detectable signal also means the formation of a colorimetric or metallic deposit (precipitate) upon a support, preferably a silver deposit.

This metallic deposit (precipitate), resulting from a chemical or biochemical reduction, preferably upon colloidal gold bound to the extremity of these detection molecules, is detected by method(s) well known by the person skilled in the art upon any type of solid support surface, including microarrays for example the one described in EP1179180B1. The use of a colorimetric detectable signal through the formation of a silver deposit upon the support is a preferred embodiment of the invention, as it allows detecting colorimetric signals on microarrays, and analyzing the data visually, without the need of a scanner or any other detection equipment.

Quantification is a preferred embodiment of the method since the level of activity of a transcriptional factor or the amount of a biomarker present in the biological sample may influence the tumor properties and so the decision on the use or not of a given anti-tumoral or prophylactic compound. Avoiding side effects is also one challenge of a therapeutic treatment which may well be achieved through quantitative measurements of the presence and/or the activity of (tumor) biomarkers.

The steps (a) to (d), possibly with steps (e) and/or (f) and possibly (g) are preferably performed upon the same (insoluble) solid support. Solid supports are made in any suitable material, such as a glass, plastic or metallic support of different formats, preferably multiwell plates, discs or slides, which are adequate for microarray detection and/or quantification and adequate for automation, especially for high throughput screening automation. The inventors have found that performing steps (a) to (d), possibly in combination with (e) and/or (f) and possibly (g) on a same solid support can lead to the establishment of a tumor profile from limited amounts of biological material, which is a hallmark of most clinical samples. Accordingly, a biopsy which is the typical source of human solid tumors, usually provides 50 to 100 mg of tissue. This corresponds to about 1000µg of total protein extract or around 150µg of nuclear extract, when a classical extraction method from snap frozen tissue is applied. This allows performing several individual assays of biomarkers, i.e. the detection of biomarkers by ELISA, or the analysis of the DNA-binding capacity of transcriptional factors by methods as the one described in the patent application WO01/73115. Taking into account the necessity of performing every experiment in triplicate and to add reference wells, which must also contain the sample, classical methods can detect a maximum of five (tumor) biomarkers, or the DNA-binding capacity of a maximum of four transcriptional factors. This is very limiting, since establishing the profile of many tumors will likely require the analysis of more (tumor) biomarkers.

The method of the present invention is not confronted to those limitations. Indeed, the inventors have found that performing multiple assays on a same solid support allows analyzing as many as 20 different biomarkers simultaneously with very limited amounts of biological materials, such as 30µg of nuclear extract or 50µg of total protein extract. Therefore, the method of the invention advantageously allows establishing a tumor profile for virtually any tumor type. Moreover, establishing a tumor profile as proposed by the present invention requires the simultaneous analysis of multiple (tumor) biomarkers, as those (tumor) biomarkers fluctuate over time with the tumor status, and should therefore be analyzed from a same biopsy, at a defined time point of the disease progression.

In a preferred embodiment of the invention, the solid support comprises specific surfaces or areas containing capture probes dedicated to a specific detection step, preferably in the form of microarrays. In this preferred embodiment, the DNA- and protein capture probes are respectively clustered at different locations on the solid support. This allows contacting them separately with their respective biomarkers under binding conditions which are optimized for each binding type. This is particularly advantageous as the optimal binding of transcriptional factors to their target DNA capture probes on one hand, and of proteins to protein capture probes on the other hand, require specific, and different, experimental conditions. Accordingly, the inventors observed that the optimal binding buffer for transcriptional factors-DNA interactions is a low salt Hepes buffer, supplemented with glycerol, a low dithiothreitol concentration and protease and phosphatase inhibitors, while the optimal binding buffer for protein-protein interactions is a MOPSO buffer containing detergents and protease inhibitors but no glycerol and no dithiothreitol.

In a preferred embodiment of the invention, the capture probes are arranged upon a solid support surface in an array format. Microarrays are preferably in the form of spotted capture probes obtained by contact or contactless methods. Each spot occupies a surface of preferably between about 1µm2 and about 10mm2, and more preferably between about 0.01 and about 0.3mm2. A typical microarray for p53 mutational analysis is the Affymetrix p53 DNA microarray, which allows detection of mutations affecting such DNA binding protein.

In a particular embodiment of the invention, the DNA capture probes and the protein capture probes are spotted as two separate microarrays on a same solid support, for performing the binding of their respective biomarkers under their respectively optimal conditions.

In one particular embodiment of the invention, the method is performed on two different solid supports, each one being specific for one type of capture probes or one type of detection. The inventors have found that the possibility to group DNA and protein capture probes on only two solid supports presents the main advantage of enabling the detection and/or the quantification of a large number of different (tumor) biomarkers in a same assay.

In another embodiment, the solid support is beads, each one bearing capture probe(s) for the binding of one (tumor) biomarker.

Multiwell plates are one of the preferred solid supports used by the invention. They preferably contain one type of capture probe per well or a mix of two or more capture probes per well, where each capture probe is dedicated for specifically binding one type of (tumor) biomarker (transcriptional factor, interacting partner or another (tumor) biomarker that is not a transcriptional factor) to be detected.

The immobilization of the capture probes is preferably obtained by a coating of a multiwell plate surface or by a spotting either on the bottom of a well from a multiwell plate or at the surface of a glass or plastic disc or slide. Immobilization can be of covalent or non covalent type, and may be performed directly on the surface of the support or on a substrate present on the surface of the support. In a preferred embodiment, the substrate is composed of streptavidin or avidin molecules. In another preferred embodiment, the substrate is composed of reactive aldehyde molecules than can react with NH₂ terminal group(s) of capture probes.

Another aspect of the present invention is related to a diagnostic, quantification and/or screening kit or device comprising means and media for performing the method according to the invention. The kit (or device) is advantageously used to provide a tumor profile from a biological sample within 24 hours, allowing the clinician to select or adapt a preferred treatment to a subject in a very short time period. This is of particular importance, as tumors are rapidly evolving diseases, which require a fast reaction from the clinician.

In a preferred embodiment of the invention, the kit (or device) comprises capture probes which are arranged in an array format. In a more preferred embodiment, the nucleic acids (DNA) capture probes and the protein capture probes are arranged in two different arrays on a same support. This is very advantageous, as it allows detecting up to 20 (tumor) biomarkers with the array containing the protein capture probes, and analyzing the DNA-binding capacity of up to 20 transcriptional factors with the array containing the (nucleic acids) DNA capture probes. All those assays require as few as about 20mg of biological sample, and the method and kit (or device) of the present invention are therefore compatible with any type of tumor, even those for which very small biopsies can be obtained, such as brain tumors.

In one particular embodiment of the invention, the kit is advantageously used as a technological platform for providing information to researchers by establishing a(n) (anti)- tumoral profile(s) of new therapeutic and/or prophylactic compounds, to screen, to select and/or to recover compounds that are more efficient at a specific step of this method and, therefore, preferably used in a specific anti-tumoral protocol affecting the tumor profile. In a preferred embodiment, the method is performed on biological samples obtained from cells in culture, on which the experimental settings are easier to define and which provide less variability than more complex tumor samples. In a more preferred embodiment, the method is performed on immortal or transformed cell lines.

In a particular embodiment, the method according to the invention is used for the screening of new (isolated or synthesized) compounds interacting preferably with some (tumor) biomarkers and corresponding to a specific (anti-)tumor profile. Compound(s) can be administered to the subject (administration can be local, i.e. by injection near or within the tumor). These compound(s) can also be contacted with the tumoral tissue, tumor cells or fluid, extracts or fractions thereof. In a particular embodiment of the invention, compound(s) is (are) added to cells in culture, or to extracts thereof.

The present invention concerns also a screening (or selection) method which comprises the steps of:
- putting into contact possible antitumoral compounds with (a) first or second (tumor) biomarker(s), and
- selecting and possibly recovering potential antitumoral compound(s) that inhibit(s) the binding between the said (tumor) biomarker(s) and its (their) capture probe(s) present upon a solid support as described in the kit (or device) according to the invention.

### Definitions

Unless otherwise defined, all technical and scientific terms used herein have the same meaning as is commonly understood by one person skilled in the art to which this invention belongs.

The term 'tumor' refers to a cell hyper proliferating symptom or disease that can be benign or malignant and can develop metastasis (Darnell, Lodish & Baltimore, Molecular cell biology.

The term 'tumor profile' according to the invention is the combined information related to the presence and activity of one or more (tumor) biomarker(s) from a specific cell or group of cells obtained from a specific subject, as well as their characteristics. Said tumor profile is important for the monitoring of a possible responsiveness of a subject's tumor following an anti-tumoral protocol, especially responses to the addition of one or more anti-tumoral compounds interacting with or targeting these (tumor) biomarkers. "Targeting" means modifying, i.e. activating or inhibiting. Targeting may occur directly through physical contact between the anti-tumoral compound and the (tumor) biomarker, or indirectly, i.e. through the action of the compound on a molecule which lies upstream from, and can modulate - either activate or inhibit - the (tumor) biomarker. Targeting also means replacing a (tumor) biomarker, i.e. through gene therapy, if the (tumor) biomarker (i.e. anti-oncogene) is found deficient.

'Biological sample' includes a biological fluid or a biological tissue (or an extract thereof). Examples of biological fluids include urine, blood, plasma, serum, saliva, semen, stool, sputum, cerebral spinal fluid, tears, mucus, amniotic fluid or the like. Biological tissues are aggregates of cells, usually of a particular kind together with their intercellular substance that form one of the structural materials of a human or animal subject, including connective, epithelium, muscle and nerve tissues. Examples of biological tissues also include organs, tumors, lymph nodes, arteries and individual cell(s). The biological fluid or tissue can be from a normal tissue or a diseased tissue. Normal tissue refers to a tissue free of disease such as tumor, metabolic disorder or immune system dysfunction. Diseased tissue refers to a pathological condition in an organism resulting e.g. from infection or genetic defect, and is characterized by identifiable (tumoral) symptoms. 'Biological samples' also include in vitro cultured cells, extracts or fractions thereof. The in vitro cultured cells include transformed cell lines or primary cells. These cells can be treated, wherein the treatment is physical, chemical and physiological or a drug administration. 'Biological sample' also encompasses a protein or a mix or proteins isolated from the biological tissue, fluid or the cellular culture referred to above, i.e. following a purification procedure.

The term 'drug' or 'anti-tumoral compound' as used herein, should be interpreted broadly and includes, but is not limited to, compounds of known efficacy and safety, drug or compound candidates picked randomly from libraries of compounds, and drugs at every level of investigation there between. Drugs include compounds which affect the level of activation of cells and include modulators of kinase/phosphatase activities, transcription factors activity, or any other (tumor) biomarker.

'Anti-tumoral therapy' refers to any type of treatment administered to a subject to treat a tumor, including but not limited to hormonal therapy, chemotherapy, radiotherapy, diet and gene therapy.

'Anti-tumoral compound' refers to any type of molecule developed with the aim of negatively affecting a tumor. An anti-tumoral compound can be of known efficacy and safety, a drug candidate picked randomly from libraries of compounds, or a drug at every level of investigation there between. An anti-tumoral compound can exert its effect by killing tumoral cells, stopping or slowing their development, proliferation or maturation.

According to the invention, the term 'biomarker(s)' corresponds to proteins, preferably regulatory proteins, such as transcriptional factors, DNA binding proteins, and other intracellular non DNA-binding proteins, or extracellular proteins, and characteristics thereof. Other types of (tumor) biomarkers are preferentially proteins related to cellular activation, transformation, and apoptosis. (Tumor) biomarkers also include complexes or associated groups or molecules, which are required for the activation status of the (tumor) biomarker(s). Said associated groups or molecules are also molecules required to obtain interaction of transcriptional factors with DNA. 'Associated groups or molecules' are elements that interact with these (tumor) biomarkers during or after their binding to a nucleic or a proteic capture probe.

As used herein, 'detecting and/or quantifying' a (tumor) biomarker or a signal are intended to include qualitative and/or quantitative determination in the sense of obtaining an absolute value for the amount or concentration or activity of the (tumor) biomarker present in the sample, and also of obtaining an index, ratio, percentage, visual and/or other value indicative of the level of the (tumor) biomarker in the sample. Assessment may be direct or indirect and the chemical species actually detected need not, of course, be the (tumor) biomarker itself, but may for example be a derivative thereof or some further substance.

According to the invention, the term 'protein' refers to a complete protein or fragment thereof, such as polypeptide, oligopeptide, etc. Fragment means portion of at least 5 consecutive amino-acids, preferably at least 25 consecutive amino-acids, more preferably at least 50 amino-acids.

'Transcriptional factors' are proteins that bind to a specific sequence of double-stranded DNA molecules, and modulate or regulate positively or negatively the transcription of DNA. Transcriptional factors are activated through different mechanisms such as translocations, post-translational modifications like phosphorylations, and association with cofactors, such as co-activators and co-repressors, which respectively increase and decrease the transcriptional regulation. The activity of a transcriptional factor refers to its capacity to modulate or regulate the transcription of DNA. The DNA binding capacity of a transcriptional factor refers to its capacity to bind a specific sequence of double-stranded DNA.

A 'protein characteristic' is any structural modification of the protein, including post-translational modification, mutation, truncation or any other modification of the proteinic sequence that results in a modification of the protein structure, activity or binding capacity to any other protein or compound.

The terms 'post-translational modification' include phosphorylation on a protein residue, being a tyrosine, serine, threonine and/or histidine, that can be detected by phosphotyrosine-specific (monoclonal) antibodies, phosphoserine-specific antibodies and phosphothreonine antibodies, for example. (Monoclonal) antibodies used to detect these modifications also include (monoclonal) antibodies specific to (a) phosphorylated residue(s) of a protein, or a fragment of the protein containing the phosphorylated residue(s).

'Interacting partner' of a transcriptional factor refers to any molecule that can physically interact, directly or indirectly, to a transcriptional factor, for obtaining its activation or for allowing its binding to a double DNA sequence.

The term 'solid support' refers to any material made of elements selected from the group consisting of polymers, glass, metals or silicium; said material being able to fix directly or be covered by a layer able to fix the capture probes necessary for the biomarkers interaction and assay. The forms of the supports are preferentially, but not limited to, multi-well plates of 96, 384 or 1536 wells, micro-arrays with planar surfaces or with cavities, where the different capture probes are present in different spots on the micro-arrays, or (possibly magnetic) microbeads for using for their assay in FACS machines or for facilitating their washing through their magnetic properties.

The terms 'capture probe(s)' refer to molecule(s) or complex (or combination) of molecules, that are capable of specifically binding, directly or indirectly, target (tumor) 'biomarkers'. Capture probes are preferably composed of, or contain DNA or proteins. DNA capture probes preferably comprise double-stranded DNA containing a site for the specific binding with transcriptional factors or other DNA binding proteins. These sequences may be naturally occurring or genetically engineered DNA sequences. The specific binding sites for the transcriptional factors or DNA binding proteins may be naturally occurring sites or consensus binding sites. Such specific binding sites can be attached to or be part of a molecule that is considered as a spacer when bound to a solid support. Protein capture probes are any proteic molecules capable of interacting with the (tumor) biomarkers or with a label bound to the (tumor) biomarkers. They are proteins such as (monoclonal or polyclonal) antibodies or hypervariable portions thereof containing various parts of the heavy and/or the light chains of the antibodies, peptides, transcriptional factors, scaffold proteins or receptors.

As used herein, 'antibody' includes immunoglobulins that are composed of two light chains and two heavy chains linked by disulfide bounds and also hypervariable portions thereof, such as Fab, (Fab)2, Fv or single variable region fragments (scFv).

As used herein, the term 'receptor' refers to a molecule that has an affinity for a given ligand. Receptors may be naturally-occurring or synthetic molecules.

'(Micro)Array' means a solid support or a substrate on which capture probes are immobilized in order to be able to bind to the given specific target (tumor) biomarkers. The array is preferentially composed of spots of capture probes deposited at a given location on the surface or within the support or on the substrate covering the support. In one particular application of this invention, arrays of capture probes are also provided on different supports as long as the different supports contain specific capture probes and may be distinguished from each other in order to be able to detect and/or quantify the specifically bound (tumor) biomarkers. This can be achieved by using a mixture of beads having particular features and being able to be recognized from each other in order to quantify the bound (tumor) biomarkers.

### Short description of the figures and tables

Table 1 presents examples of proteins that can be considered as (tumor) biomarkers in the present invention.

Table 2 presents examples of proteins that can be considered as biomarkers of breast tumors by the present invention.

Table 3 presents examples of biomarkers associated to the p53 status in the present invention.

Table 4 presents signaling pathways containing proteins that can be considered as biomarkers by the present invention.

Figure 1 describes the procedure to analyze hierarchically the results of the method performed as in example 1.

Figure 2 describes the procedure to determine an adequate treatment of a tumor based on its p53 status, according to the method performed as in example 2.

### Examples

The following examples are provided for illustrative purpose only, and are not intended to limit the scope of the invention.

### 1. General procedure:

### A. Immobilization of capture probes on a solid support

Functionalized slides (Diaglass, EAT, Namur, Belgium) or 96well plates are used in the examples. Two types of capture probes are used: double-stranded DNA capture probes to bind transcriptional factors, and protein capture probes (antibodies) to bind both transcriptional factors and other proteins. Capture probes contain an amine group and can bind to a solid support containing aldehyde groups, except for DNA capture probes that bind to multiwell plates, which contain biotin groups and bind to wells containing streptavidin groups.

Capture probes are spotted in triplicates on the surface of glass slides as follows: DNA capture probes are dissolved in a citrate buffer supplemented with salt and detergent. Protein capture probes are dissolved in a borate buffer supplemented with detergent and molecules to preserve the activity of the capture probes (such as glycerol and/or sugar molecules, i.e. trehalose). Spotting concentrations range respectively from 5 to 3000nM for DNA capture probes, and from 0.1 to 1mg/ml for protein capture probes. Spotting is performed under controlled conditions of humidity and temperature, using a split pin or a pin and ring or a plain pin. The spots of DNA capture probes and the spots of protein capture probes are respectively clustered at spatially different places on the slides. After spotting, slides are washed, dried and 8-well SecureSeal hybridization chambers (Grace-Biolabs) are apposed onto the slides to physically isolate the DNA from the protein capture probes, and allow subsequent separate incubations.

When multiwell plates are used, DNA and protein capture probes are dissolved in a phosphate buffer supplemented with salt. DNA capture probes are biotinylated, and coated on streptavidin coated multiwells. Coating concentrations usually range respectively from 10 to 100pmoles/ml for DNA capture probes, and from 0.1 to 10ng/µl for protein capture probes. Coating is performed by incubating the plates 1h at 37°C for DNA capture probes, and overnight at 4°C for protein capture probes, followed by washing.

### B. Blocking

Blocking is performed in a phosphate buffer with salt and a blocking protein (such as BSA or dried milk). When protein capture probes are bound to multiwell plates, the blocking solution is supplemented with sucrose. Blocking is performed for one hour at RT, followed by washing.

### C. Addition of samples to the chambers/wells

The sample preparation must be optimized for each sample type. Nucleic protein extracts from the sample to be analyzed are added to the supports containing DNA capture probes, while cytoplasmic or total protein extracts, or proteins from extracellular sources, are added to the supports containing protein capture probes. Incubation is performed in an adequate binding buffer (EAT, Namur, Belgium), which differs fro transcriptional factors and proteins binding, for 1 to 16 hours under gentle agitation. Wells are emptied and washed.

### D. Detection with antibodies

Primary antibodies specific to each biomarker to be detected are added to the corresponding chambers/wells in a phosphate buffer supplemented with salt and a blocking protein. Incubation is performed for 1h at room temperature, followed by washing. Labeled secondary antibodies are then added for 1h at room temperature, followed by washing. If slides are used, fluorescently-labeled secondary antibodies are used; if multiwell plates are used, HRP-conjugated secondary antibodies are used.

If slides are used, hybridization chambers are removed, and slides are washed and dried. A fluorescence detection of signals is performed by scanning slides using a laser confocal scanner "ScanArray" (Packard, USA) at a resolution of 10 µm. If multiwell plates are used, signal measurement is performed by adding 100µl/well tetramethylbenzidine (Biosource, Belgium) for 10 minutes in obscurity and 100µl/well stop solution (Biosource, Belgium), followed by OD measurement at 450nm.

### 2. Example 1: method to profile breast tumors for their responsiveness to different drugs

Two microarrays are spotted on glass slides: a first microarray is composed of triplicate spots of a double-stranded DNA capture probe which contain a binding site specific for ERα. The sequence of the sense strand is the following: 5'-CAGGTCACAGTGACCTGATCAAAGTT-3'. A second microarray is composed of triplicate spots of protein capture probes corresponding to antibodies specific respectively for Erα, ErbB1, ErbB2 and mTOR. Hybridization chambers are attached to the glass slides to physically separate the two microarrays.

The first microarray is contacted with a protein extract from a breast tumor sample diluted in a binding buffer composed of Hepes buffer supplemented with salt, EDTA, glycerol, protease and phosphatase inhibitors. The second microarray is contact with a protein extract from the same breast tumor sample diluted in a MOPSO buffer supplemented with salt, detergents, and protease inhibitors. Incubation is performed for one hour at 21°C under gentle agitation in a Thermomixer (Eppendorf, Germany). Samples are removed and microarrays are washed.

An antibody specifically recognizing the activated form of ERα is contacted with the first microarray. A mix of antibodies, each specifically recognizing one of the proteins ERα, ErbB1, ErbB2 and mTOR, is contacted with the second microarray, followed by washing. Each microarray is then contacted with Cy3-labeled secondary antibodies under indirect light, followed by washing. Hybridization chambers are removed; slides are rinsed twice in water, dried and scanned.

The signals corresponding to the triplicate spots are averaged. Five values are then obtained, informing about:
a: the presence of ERα (second microarray; ERα specific spots): signal #1
b: the DNA-binding activity of ERα (first microarray; ERα specific spots): signal #2
c: the presence of ErbB1 (second microarray; ErbB1 specific spots): signal #3
d: the presence of ErbB2(second microarray; ErbB2 specific spots): signal #4
e: the presence of mTOR (second microarray; mTOR specific spots): signal #5.

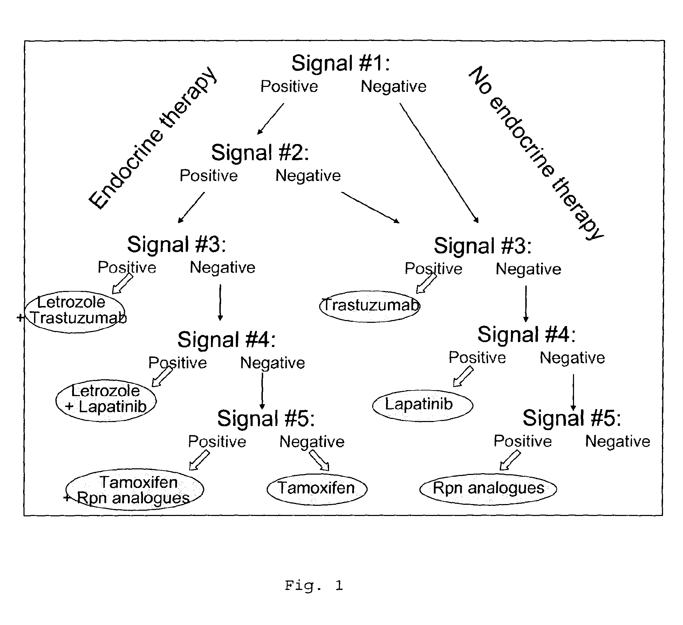

The following procedure, depicted in figure 1, is then applied to hierarchically analyze the data. Some molecules are proposed at different steps of the analysis procedure for illustrative purposes only. They are in no way intended to orient the choice of the clinician, or to exclude other molecules, and should therefore be understood as examples only.

Signals #1 and 2 respectively inform about the presence and activity of ERα. An active estrogen receptor is a prerequisite to successfully apply an endocrine treatment. Both signals must then be positive. If a positive signal #1 is observed, the signal #2 must be analyzed. A positive signal #2 indicates that an endocrine therapy is appropriate, such as tamoxifen or letrozole, while a negative signal #2 indicates that an endocrine therapy will probably be ineffective.

The most adapted molecule to be prescribed in an endocrine therapy depends on the overexpression of an ErbB member by the tumor. The simultaneous presence of ERα and ErbB is better treated by the use of letrozole (Femara® ) than tamoxifen. This information is accessible through the analysis of signals #3 and 4. Therefore, if at least one of the signals #3 and 4 is positive, letrozole might be proposed to replace tamoxifen. Moreover, some molecules are more potent to inactivate both ErbB1 and ErbB2 than others. Hence, the simultaneous presence of both receptors is better treated by the use of inhibitors such as Lapatinib (Glaxo SmithKline), while Trastuzumab (Herceptin® ) is argued to be an incompletely effective anti-ErbB targeting agent. Therefore, if both signals #3 and 4 are positive, the use of molecules such as Lapatinib may be recommended.

Resistance often occurs in absence of any evidence for ErbB member expression. Signal #5 analyzes the presence of a downstream target of this signaling cascade: mTOR. If signals #3 and 4 are negative, indicating that there is no evidence for an ErbB-mediated resistance, the presence of the downstream target mTOR is analyzed: a positive signal #5 points to the use of a rapamycin analogue.

### 3. Example 2: method to profile tumors for a tailored p53 treatment

Individual wells of a 96well plate are coated with different capture molecules: a first series of 5 wells are coated with a monoclonal antibody to p53; a second series of 5 streptavidin-coated wells are coated with a biotinylated double-stranded DNA capture probe which contains a binding site specific for p53. The sequence of the sense strand is the following: 5'-CTTGGACATGCCCGGGCATGTCCCTC-3'; a third series of 5 wells are coated with a monoclonal antibody to Mdm2; a fourth series of 5 wells are coated with the antibody to p53, as used in the first series.

Two wells of each series are contacted with the binding buffer only ('blank'), and the left three wells of each series are contacted with a protein extract from the test sample diluted with the binding buffer ('test'). The binding buffer used for series 1, 3 and 4 is composed of a MOPSO buffer containing salt, detergents, proteins and protease inhibitors. The binding buffer used for series 2 is a Hepes buffer containing salt, glycerol, EDTA, DTT, protease and phosphatase inhibitors. Incubation is performed for one hour at 21°C under gentle agitation. Samples are removed and wells are washed.

Polyclonal antibodies specifically recognizing p53 (series 1 and 2) or Mdm2 (series 3 and 4), are contacted with the corresponding wells, followed by washing.

Each well is then contacted with a HRP-conjugated secondary antibody specific for the polyclonal antibody used at the preceding step, followed by washing and colorimetric detection.

For each series of wells, the signals corresponding to the 'blanks' are averaged and deduced from the average value of the 'tests'.
Four values are then obtained, informing about:
a: the presence of p53: signal #1
b: the DNA-binding activity of p53: signal #2
c: the presence of Mdm2: signal #3
d: the interaction of p53 and Mdm2: signal #4.

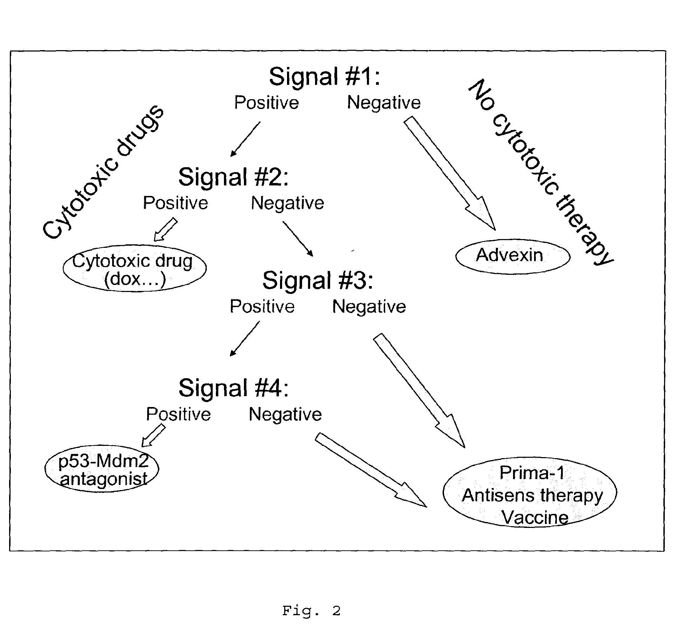

The following procedure, depicted in figure 2, is then applied to hierarchically analyze the data. Some molecules are proposed at different steps of the analysis procedure for illustrative purposes only. They are in no way intended to orient the choice of the clinician, or to exclude other molecules, and should therefore be understood as examples only.

An absence of signal #1 indicates that p53 is not present in the biological sample. The use of a gene therapy strategy can then be envisioned, i.e. through the adenoviral delivery of a wild type p53 gene (ex: Advexin® ).

Signals #1 and 2 respectively inform about the presence and DNA-binding activity of p53. An active apoptotic cascade is a prerequisite to successfully apply a cytotoxic therapy, using drugs like doxorubicin (Adriamycin® ). Both signals must then be positive. If a positive signal #1 is observed, the signal #2 must be analyzed. A positive signal #2 indicates the use of a cytotoxic drug, while a negative signal #2 indicates that the p53 protein is present but defective. This claims for a further analysis of the remaining 2 signals.

Signal #3 informs about the presence of Mdm2, which is known to sequester p53, preventing its activity. If signal #3 is positive, signal #4 must be analyzed. A positive signal #4 indicates an interaction between p53 and Mdm2. These points to the use of small antagonists of this p53-Mdm2 interaction as therapeutic molecules.

### TABLES

**Table 1: examples of proteins whose detection and/or measurement of one characteristic can be considered as biomarkers in the present invention**

| Transcriptional factors: | Other regulatory proteins: |
|---|---|
| p53, cMyc, ER, PR, AR, SMAD4, HIF1α, NFκB | ErbB1, ErbB2, mTOR, KAI1/CD82, cKit, AIB-1, p21, Ki67, GSTP1-1, Ras, p14/ARF, Mdm2, Mdmx, COP1, Pirh2, E6AP, CBP, p300, BRCA1, BRCA2, ATM, CHK2, VEGF, bFGF, uPA, Bcl-2, MDR-1 |

**Table 2: examples of proteins whose detection and/or measurement of one characteristic can be considered as biomarkers of breast tumours by the present invention**

| Transcriptional factors and other regulatory proteins | Drugs targeting these molecules |
|---|---|
| ERα | Tamoxifen, letrozole |
| PR | |
| Stat3, Stat5 | |
| HIF1α | PX-478 |
| ErbB2 | Trastuzumab |
| ErbB1 (EGFR) | ZD1839, lapatinib |
| AIB1 | |
| G proteins | Farnesyltransferase inhibitors |
| mTOR | Rapamycin analogues |
| BRCA1 | |
| BRCA2 | |
| HDAC6 | |
| NCoR | |

**Table 3: examples of biomarkers associated to the p53 status in the present invention**

| Characteristic of p53 to be detected | Biomarker | Example of therapeutic action | Example of drug |
|---|---|---|---|
| p53 absence | p53 | gene therapy | Ad-p53 (Advexin, RHC58500) |
| p53 presence | p53 | Different options | |
| p53 DNA-binding capacity | DNA-bound p53 | Use cytotoxic drugs | Doxorubicin, cisplatin, paclitaxel, ... |
| Absence of p53 DNA- binding capacity | DNA-bound p53 | Vaccine, antisens oligo | EL625 |
| p53 mutations within the DNA-binding site | Mutated p53 | molecules modifying the p53 conformation | CP31398, Prima-1 |
| p53 interaction with Mdm2 | Mdm2; p53-bound Mdm2 | Antagonists of the p53-Mdm2 interaction | Nutlins |
| p53 interaction with Mdmx | Mdmx; p53-bound Mdmx | Antagonists of the p53-Mdmx interaction | |
| p53 interaction with Pirh2 | Pirh2; p53-bound Pirh2 | Antagonists of the p53-Pirh2 interaction | |
| p53 interaction with Cop1 | Cop1; p53-bound Cop-1 | Antagonists of the p53-Cop1 interaction | |

**Table 4: signalling pathways containing proteins that can be considered as biomarkers within the present invention**

| Signalling Pathway | Main proteins involved |
|---|---|
| PI3K/Akt pathway | Akt, Forkhead, FOXO |
| Cell cycle pathway | Bcl-2, Bcl-xL |
| Apoptotic pathway | Bad, Bcl-2, p53, Mdm2 |
| EGFR (ErbB1) pathway | ErbB1, mTOR |
| ErbB2 pathway | ErbB2, mTOR |
| MAPK pathway | Ras, Raf, cMyc |
| NFκB pathway | NFKB, Bcl-xL |
| Angiogenesis | VEGF, PDGF, TSP-1, EGFR, bFGF, EGF, PlGF, HGF, IL-6, IL-8, IL-12, Ang1, Ang2, angiogenin, angiostatin, endostatin, TSP1, MMPs |

## Claims

1. A method for providing a tumor profile of a biological sample obtained from a tumoral tissue, tumor cells or fluid of a subject, preferably a human subject, by a detection and/or quantification of (tumor) biomarkers possibly present in the sample, said method comprising at least the steps of:
a. contacting said biological sample with capture probes,
b. detecting and/or quantifying a first (tumor) biomarker being a transcriptional factor in the biological sample after binding to a first capture probe,
c. detecting and/or quantifying the DNA-binding capacity of the said transcriptional factor in the biological sample after binding to a second capture probe, which possibly differs from the first capture probe and contains a double-stranded DNA sequence specifically binding said transcriptional factor, and
d. detecting and/or quantifying a second (tumor) biomarker different from a transcriptional factor in the biological sample after binding to a third capture probe,
wherein the capture probes are bound directly or indirectly to a solid support, and wherein only the overall integration of the results obtained at each step following step (a) provides information on the tumor profile.

2. The method according to the claim 1,
wherein the steps are successive steps.

3. The method according to the claim 1,
wherein the successive steps are steps (a), (d), (b) and (c) .

4. The method according to the any of the preceding claims, which further comprises the step of:
e. detecting and/or quantifying a modification of the said transcriptional factor affecting its activity.

5. The method according to any of the preceding claims, which further comprises the step of:
f. detecting and/or quantifying the binding capacity of the said transcriptional factor with interacting partner(s), and
g. possibly detecting these associated molecules in the biological sample.

6. The method according to any of the preceding steps, where the information on the tumor profile allows the selection of one or more adequate anti-tumoral compound(s), preferably compounds targeting detected (tumor) biomarker(s), these compounds being administered to the subject from whom the biological sample has been obtained.

7. The method according to any of the preceding claims, wherein the step (b) of detecting and/or quantifying a first biomarker being a transcriptional factor and the step (d) of detecting and/or quantifying a second biomarker, are obtained by capture probes bound to a solid support and interacting specifically with said transcriptional factor or said second biomarker, and wherein the obtained interactions result in detectable signals.

8. The method according to the claim 7,
wherein the capture probes (monoclonal) are antibodies or hypervariable portions thereof.

9. The method according to the claim 7,
wherein the capture probes are receptors.

10. The method according to any of the preceding claims, wherein the step (c) of detecting and/or quantifying the DNA-binding capacity of the transcriptional factor in the biological sample is obtained by a binding of the transcriptional factor to a capture probe bound to a solid support and which contains a double-stranded DNA sequence specifically binding said transcriptional factor, and wherein the obtained binding results in a detectable signal.

11. The method according to claim 10, wherein the double-stranded DNA sequence is fixed upon the solid support surface by a spacer having a length of at least about 6.8 nm.

12. The method according to any of the preceding claims, wherein the step (e) of detecting and/or quantifying a modification of the transcriptional factor affecting its activity is obtained by binding of the said modified transcriptional factor to a capture probe, specifically binding the said modified transcriptional factor and wherein the obtained binding results in a detectable signal.

13. The method of claim 12, wherein the capture probe is a double-stranded DNA sequence.

14. The method of claim 12, wherein the capture probe is protein.

15. The method of claim 12, wherein the protein is a (monoclonal) antibody or a hypervariable portion thereof.

16. The method of any of the claims 12 to 15,
wherein the modification is a post-translational modification.

17. The method of claim 16, wherein the post-translational modification is a phosphorylation or an acetylation.

18. The method of any of the claims 12 to 15,
wherein the modification is a mutation or a truncation.

19. The method of any of the claims 12 to 15,
wherein the modification is a conformational change.

20. The method of any of the claims 12 to 15,
wherein the modification is a binding to a cofactor.

21. The method of claim 20, wherein the cofactor is a coactivator or a corepressor.

22. The method according to any of the preceding claims, wherein the step (f) of detecting and/or quantifying the binding capacity of the transcriptional factor with interacting partner(s) is obtained by a binding of the transcriptional factor and its interacting partner(s) to a capture probe specifically binding the transcriptional factor and wherein the obtained binding results in a detectable signal.

23. The method of claim 22, wherein the capture probe contains a double-stranded DNA sequence specifically recognizing the transcriptional factor.

24. The method of claim 22, wherein the capture probe is an (monoclonal) antibody or a hypervariable portion thereof specifically binding the transcriptional factor.

25. The method of claims 22 to 24, wherein the step(s) of detecting of the interacting partner(s) is obtained by a binding of a capture probe thereof recognizing the interacting partner(s) and wherein the obtained binding results in a detectable signal.

26. The method according to any of the preceding claims 7 to 25, wherein the detectable signal is obtained by a labeled detection molecule (preferably labeled (monoclonal) antibodies or a hypervariable portion thereof) that recognize the (tumor) biomarkers bound to capture probes.

27. The method according to any of the claims 7 to 26, wherein the detectable signal is a non-radioactive signal, preferably a signal selected from the group consisting of fluorescent signal, colorimetric signal, chemoluminescent signal, bioluminescent signal, radioactive signal, electronic signal and magnetic signal.

28. The method according to the claim 27,
wherein the colorimetric signal results from a metallic deposit, preferably a silver deposit upon a surface of a solid support.

29. The method according to any of the preceding claims, wherein the transcriptional factor is selecting from the group of nuclear receptors, products of oncogenes and tumor suppressor genes.

30. The method according to any of the preceding claims, wherein the second (tumor) biomarker different from a transcriptional factor is a protein involved in proliferation or apoptotic cascades.

31. The method according to any of the preceding claims, wherein the second (tumor) biomarker different from a transcriptional factor is a kinase or a phosphatase.

32. The method according to any of the preceding claims, wherein the second (tumor) biomarker different from a transcriptional factor is an extra-cellular protein.

33. The method of claim 32, wherein the extra-cellular protein is cytokine or a growth factor.

34. The method of claim 32, wherein the extra-cellular protein is an angiogenic or anti-angiogenic protein.

35. The method according to the claim 34,
wherein the angiogenic or anti-angiogenic protein is selected from the group consisting of VEGF, PlGF, PDGF, bFGF, EGF, HGF, TGFβ, IL-6, IL-8, IL-12, angiopoietin-1, angiopoietin-2, angiogenin, angiostatin, endostatin, thrombospondin-1, matrix metalloproteinases, or a mixture thereof.

36. The method according to any of the preceding claims wherein the (tumor) biomarkers are selected from those listed in table 1.

37. The method according to any of the preceding claims wherein the (tumor) biomarkers are selected from those listed in table 2.

38. The method according to any of the preceding claims wherein the (tumor) biomarkers are selected from those listed in table 3.

39. The method according to any of the preceding claims wherein the (tumor) biomarkers are selected from those listed in table 4.

40. The method according to any of the preceding claims, wherein the solid support consists of glass, plastic or metallic material.

41. The method according to any of the preceding claims, wherein the solid support has a multiwell plate format.

42. The method according to the claim 41,
wherein a capture probe is present in one well of the multiwell plate or a mix of at least two capture probes is present in one well of the multiwell plate.

43. The method according to any of the preceding claims, wherein the solid support has a disc format or a slide format.

44. The method of any of the claims 41 to 43,
wherein the capture probes are arranged in an array format.

45. The method of claim 46, wherein the capture probes are composed of nucleic acids (DNA) capture probes and protein capture probes, and wherein the nucleic acids (DNA) capture probes and the protein capture probes are spotted in two separate arrays upon the solid support.

46. The method according to any of the preceding claims, wherein the capture probes are bound to two (different) solid supports.

47. The method of claim 46, wherein the nucleic acids (DNA) capture probes are bound to a first solid support and wherein the protein capture probes are bound to a second solid support.

48. The method according to any of the preceding claims, where the capture probes are bound to more than two solid supports.

49. A diagnostic, quantification and/or screening kit or device comprising means and media for performing the method according to any of the preceding claims and comprising capture probes bound directly or indirectly to one or more solid supports and interacting specifically with biomarkers specific of a tumor profile.

50. The kit or device according to the claim 49, which comprises two types of capture probes comprising nucleic acids (DNA) capture probe(s) interacting specifically with a first (tumor) biomarker(s) being (a) transcriptional factor(s) and) protein capture probe(s) interacting specifically with a second (tumor) biomarker(s) that is (are) any protein(s) including (excluding) transcriptional factor(s).

51. The kit or device according to the claim 47, wherein the two types of capture probes are bound to spatially different locations on the solid support.

52. The kit or device according to the claim 50 or 51, wherein the two types of capture probes are bound to the support in the form of array(s).

53. The kit or device according to the claims 49 to 52, which further comprises means and/or media for detecting and/or recording (a) detectable signal(s) resulting from the binding between capture probes and (tumor) biomarkers.

54. A method for the screening of anti-tumoral compounds which comprises the steps of adding said anti-tumoral compounds with the tumor biomarkers bound to capture probes and present in the diagnostic quantification and/or screening kit or device according to the claims 50 to 53, characterizing anti-tumoral compounds able to interact with one or more of these tumor biomarkers, according to a specific tumoral profile and possibly recovering the said **characterized** anti-tumoral compounds.

55. The method according to the claim 54, which comprises the step of recovering anti-tumoral compounds that inhibit the binding between one or more biomarkers and their capture probes.

56. A method of treatment of a subject suffering from a tumor which comprises the steps of :
- adding to said subject suffering from said tumor, a sufficient amount of a pharmaceutical composition comprising an adequate pharmaceutical carrier and one or more anti-tumoral compounds selected by the method according to the claim 6 or recovered by the method of claims 54 or 55.


SEQUENCE LISTING

<110>  Eppendorf Array Technologies SA

<120>  Method and kit to profile tumors by biomarker analyses including
       transcriptional factor assays

<130>  BP.EATE.005A/EP

<160>  2

<170>  PatentIn version 3.3

<210>  1
<211>  26
<212>  DNA
<213>  Artificial Sequence

<220>
<223>  ERalpha DNA capture probe (sense strand, 5'-3')

<400>  1
caggtcacag tgacctgatc aaagtt                                          26


<210>  2
<211>  26
<212>  DNA
<213>  Artificial Sequence

<220>
<223>  p53 DNA capture probe (sense strand, 5'-3')

<400>  2
cttggacatg cccgggcatg tccctc                                          26